# FASCICULE DE BREVET EUROPEEN

(11) **EP 0 622 087 B1**
(45) Date de publication et mention de la délivrance du brevet: **20.08.1997**
(21) Numéro de dépôt: 94420131.8
(22) Date de dépôt: 27.04.1994
(51) Int. Cl.: A61M 1/34

(54) **Procédé et dispositif d'injection d'un liquide stérile et apyrogène obtenu par filtration**
Vorrichtung und Verfahren zur Injektion einer durch Filtration pyrogenfrei und steril hergestellten Flüssigkeit
Process and device for injecting a sterile and pyrogenfree fluid obtained by filtration

(30) Priorité: 27.04.1993 FR 9305198
(43) Date de publication de la demande: 02.11.1994
(73) Titulaire: HOSPAL INDUSTRIE, F-69883 Meyzieu Cédex (FR)
(72) Inventeur: Simard, Laurent, F-69007 Lyon (FR)

(56) Documents cités:
- EP-A- 0 121 085
- EP-A- 0 212 127
- EP-A- 0 270 794
- EP-A- 0 571 303
- WO-A-91/05576
- US-A- 4 702 829
- US-A- 5 069 792
- PATENT ABSTRACTS OF JAPAN vol. 13, no. 211 (C-597)17 Mai 1989 & JP-A-10 030 603 (KAYABA JUN) 1 Février 1989
- PATENT ABSTRACTS OF JAPAN vol. 12, no. 491 (C-554)21 Décembre 1988 & JP-A-63 205 104 (KUSADA MASATAKA) 24 Août 1988

## Description

La présente invention concerne un dispositif d'injection d'un liquide stérile et apyrogène obtenu par filtration et un procédé de contrôle de l'intégrité du filtre utilisé à cette fin.

Le dispositif d'injection selon l'invention est du type comprenant :
- au moins un filtre ayant un premier et un second compartiments séparés par une membrane poreuse, le premier compartiment ayant une entrée connectable à une source de liquide à filtrer et le second compartiment ayant une sortie connectée à une canalisation d'injection ;
- des moyens pour faire circuler le liquide au travers du filtre et dans la canalisation d'injection ; et
- des moyens de vérification de l'intégrité du filtre.

Un tel dispositif est décrit dans le brevet US 4 702 829, en combinaison avec une installation d'hémodiafiltration. Dans ce dispositif, le premier compartiment d'un premier filtre est muni d'un évent isolé de l'atmosphère par un microfiltre hydrophobe et il est relié, par l'intermédiaire d'un second filtre, à une canalisation d'alimentation d'un circuit de dialyse. En fonctionnement, la canalisation d'injection est connectée à un piège à bulle d'une canalisation de retour de sang épuré reliée à un patient.

L'intégrité des filtres est contrôlée en dehors des périodes de fonctionnement de l'installation. Pour ce faire, le dialyseur est enlevé du circuit de liquide de dialyse et le circuit de liquide de dialyse est bouclé sur lui-même de façon à former un circuit fermé. Par ailleurs, la canalisation d'injection est connectée au circuit de liquide de dialyse. On pompe alors du liquide de dialyse hors du circuit de liquide de dialyse de sorte que de l'air pénètre, par l'évent du premier filtre, dans les compartiment des filtres qui sont en communication. Le pompage est poursuivi jusqu'à ce que les compartiments des filtres qui sont en communication soient remplis d'air et qu'une dépression déterminée soit créée dans le circuit. On mesure la pression dans le circuit : si la membrane des filtres est intacte, l'air ne peut la traverser et la pression reste constante.

Ce procédé de contrôle de l'intégrité des filtres présente l'inconvénient de ne pouvoir être mis en oeuvre qu'en dehors des séances de traitement. Par ailleurs il requiert l'utilisation d'un filtre particulier ayant un premier compartiment muni d'un évent isolé de l'atmosphère par un microfiltre hydrophobe.

L'invention a pour but de réaliser un dispositif d'injection du type mentionné plus haut dont le filtre puisse être contrôlé en cours d'utilisation. Pour atteindre ce but, on prévoit, selon l'invention, des moyens de vérification de l'intégrité du filtre comprenant :
- un filtre secondaire apte à retenir bactéries et pyrogènes disposé sur la canalisation d'injection, le filtre secondaire ayant une surface de membrane suffisamment réduite pour qu'une rupture du filtre principal ait pour conséquence un colmatage rapide du filtre secondaire et
- des moyens pour détecter un encrassement du filtre secondaire pendant l'injection de liquide.

Selon une caractéristique de l'invention, les moyens pour détecter un encrassement du filtre secondaire comprennent :
- des moyens pour mesurer une différence des pressions existant de part et d'autre du filtre secondaire,
- des moyens pour comparer la différence des pressions mesurées à une valeur de seuil et, éventuellement,
- des moyens pour émettre une alarme lorsque la différence des pressions mesurées atteint une valeur de seuil.

L'invention a également pour objet un procédé de contrôle de l'intégrité d'un filtre principal utilisé pour rendre stérile et apyrogène un liquide destiné à être injecté en continu à un patient, le filtre principal ayant un premier et un second compartiments séparés par une membrane poreuse, et le premier compartiment ayant une entrée connectable à une source de liquide à filtrer et le second compartiment ayant un sortie connectée à une canalisation d'injection, caractérisé en ce qu'il comprend les étapes de :
- placer un filtre secondaire sur la canalisation d'injection, la surface de la membrane du filtre secondaire étant choisie suffisamment réduite pour qu'une rupture du filtre principal ait pour conséquence un colmatage rapide du filtre secondaire, et
- mesurer dans la canalisation d'injection, au cours de l'injection, la valeur d'un paramètre susceptible d'être influencé par un encrassement du filtre secondaire, tel que la différence des pressions existant de part et d'autre du filtre secondaire.

Selon une caractéristique de l'invention, le procédé comporte en outre l'étape de comparer la valeur mesurée du paramètre à une valeur de seuil. La valeur de seuil peut être choisie par exemple en fonction du débit ou de la température du liquide d'injection ou de ces deux caractéristiques. Par exemple, la valeur de seuil peut aussi être choisie sensiblement égale à la valeur du paramètre mesuré au début de l'injection du liquide.

Selon une autre caractéristique de l'invention, le procédé comporte en outre l'étape d'émettre une alarme ou d'arrêter l'injection lorsque la valeur mesurée du paramètre atteint une valeur de seuil.

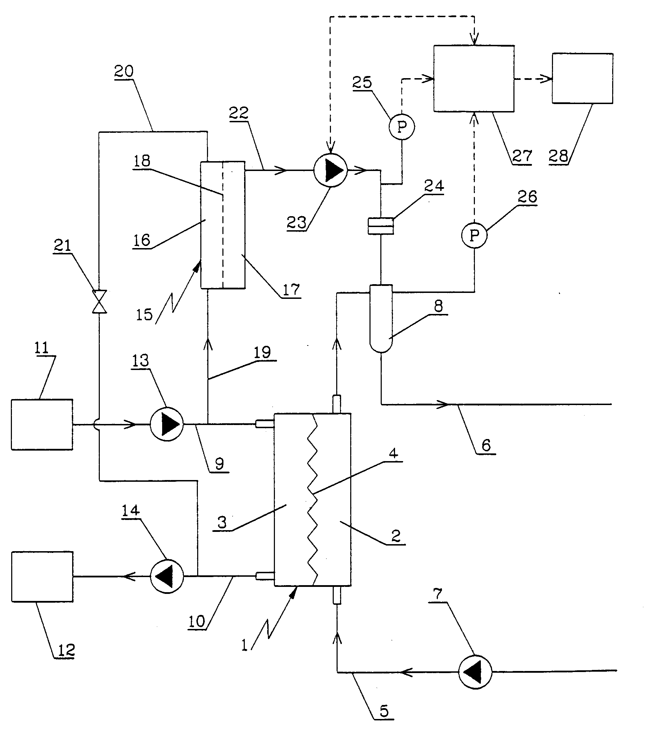

D'autres caractéristiques et avantages de l'invention apparaîtront à la lecture de la description qui va suivre. On se reportera à la figure annexée qui représente le schéma simplifié d'une installation d'hémodiafiltration comprenant le dispositif d'injection selon l'invention.

L'installation d'hémofiltration représentée sur la figure comporte un échangeur ou hémodiafiltre 1 divisé en deux compartiments 2, 3 par une membrane semi-perméable 4 permettant la dialyse et l'ultrafiltration du sang. Le compartiment 2 constitue une portion d'un circuit extracorporel de sang comprenant une canalisation de prélèvement 5 destinée à conduire le sang du patient (non représenté) à l'hémodiafiltre 1, ainsi qu'une canalisation de retour 6 destinée à conduire le sang traité de l'hémodiafiltre 1 au patient. La canalisation de prélèvement 5 est munie d'une pompe de circulation 7. La canalisation de retour 6 est munie d'un piège à bulles 8.

Le compartiment 3 est destiné à la circulation d'un liquide de dialyse. Ce compartiment a une entrée reliée, par une canalisation d'alimentation 9 munie d'une première pompe de circulation 13, à un générateur 11 de liquide de dialyse. Le compartiment 3 a une sortie reliée a une évacuation 12 de liquide usé par une canalisation d'évacuation 10 munie d'une seconde pompe de circulation 14.

L'installation d'hémodiafiltration qui vient d'être décrite est équipée d'un dispositif d'injection de liquide stérile et apyrogène obtenu par filtration du liquide de dialyse. Ce dispositif comprend un filtre principal 15 ayant un premier et un second compartiments 16, 17 séparés par une membrane poreuse 18 apte à retenir les éléments bactériens et pyrogènes du liquide de dialyse. Le premier compartiment 16 a une entrée reliée à la canalisation d'alimentation 9 de liquide de dialyse par une canalisation 19 connectée à la canalisation d'alimentation 9 entre la première pompe 13 et l'hémodiafiltre 1. Le premier compartiment 16 a une sortie reliée à la canalisation d'évacuation 10 de liquide de dialyse par une canalisation 20 connectée à la canalisation d'évacuation 10 entre l'hémodiafiltre 1 et la seconde pompe de circulation 14. Sur la canalisation 20 est disposée une vanne 21 dont l'ouverture périodique permet le nettoyage de la membrane 18 par balayage tangentiel de la surface de la membrane au moyen du liquide non filtré.

Le second compartiment 17 du filtre principal 15 a une sortie reliée, par une canalisation d'injection 22, au piège à bulles 8 de la canalisation de retour 6 du circuit extracorporel de sang. Une pompe de circulation 23 est disposée sur la canalisation d'injection 22.

Conformément à l'invention, le dispositif d'injection comprend des moyens permettant de vérifier l'intégrité du filtre principal 15 pendant la séance d'hémodiafiltration. Ces moyens de vérification comprennent un filtre secondaire 24 apte à retenir les bactéries et les éléments pyrogènes, disposé sur la canalisation d'injection entre la pompe d'injection 23 et le piège à bulles 8, et deux capteurs de pression 25, 26 connectés à la ligne d'injection 22 de part et d'autre du filtre secondaire 24. Les signaux des deux capteurs de pression 25, 26 sont fournis à une unité de commande 27, qui calcule soit en continu soit périodiquement une valeur de pression différentielle et la compare à une valeur de seuil. Si la membrane 18 du filtre principal 15 est endommagée initialement ou qu'elle se rompe en cours d'utilisation du filtre, des bactéries et des éléments pyrogènes passeront dans la ligne d'injection 22 et seront arrêtés par le filtre secondaire 24. Cet encrassement se traduira par une augmentation de la différence des pressions de part et d'autre du filtre secondaire 24. Quand cette différence atteint la valeur de seuil préalablement fixée, l'unité de commande déclenche des moyens d'alarme 28 et commande l'arrêt de la pompe 23. L'émission de l'alarme et l'arrêt de la pompe d'injection 23 peuvent aussi être commandés à deux valeurs de seuil différentes.

Le filtre secondaire 24 a une membrane dont la porosité est inférieure à environ 0,45 µm et est de préférence comprise entre environ 20 µm et environ 24 µm. La surface de la membrane est choisie en fonction du débit d'injection, qui peut être compris entre environ 1 et environ 50 millilitres par minute. Il s'agit que le filtre secondaire 24 ne provoque pas une perte de charge trop importante dans la ligne d'injection et offre simultanément une surface de membrane suffisamment réduite pour qu'une rupture du filtre principal 15 ait pour conséquence un colmatage rapide du filtre secondaire 24.

A titre d'exemple, pour un débit de l'ordre de 1 millilitre par minute, on peut utiliser un filtre de 13 millimètres de diamètre et pour un débit d'environ 20 millilitres par minute, on peut utiliser un filtre de 25 millimètres de diamètre (filtres Millex GV, fabriqué par la société Millipore, dont la membrane hydrophile de PVDF a une porosité de 0,22 µm).

Pour une taille de filtre donné, la valeur de seuil mentionnée plus haut est choisie en fontion du débit du liquide et, éventuellement, de sa température, c'est-à-dire aussi de sa viscosité. A titre d'exemple, on peut adopter comme valeur de seuil la différence des pressions existant de part et d'autre du filtre secondaire lorsque l'injection débute.

Le fonctionnement du dispositif qui vient d'être décrit est le suivant. Après connection du patient au circuit extracorporel de sang, la pompe 7 est mise en marche et le sang prélevé en continu au patient circule dans l'hémodiafiltre 1 au contact de la membrane 4. Le liquide de dialyse, préparé par le générateur de liquide dialyse 11, est mis en circulation dans les canalisations 9, 10 et le compartiment 3 de l'hémodiafiltre 1 au moyen des pompes 13, 14. A l'intérieur de l'hémodiafiltre 1, des échanges ont lieu au travers de la membrane semi-perméable 4 entre le sang et le liquide de dialyse. Ces échanges sont dus en premier lieu au phénomène de diffusion résultant de la différence entre les concentrations des solutés dans le sang et le liquide de dialyse, le liquide de dialyse étant notamment dépourvu des substances dont on veut épurer le sang (urée, créatinine). En outre, des échanges sont dus au phénomène de convection, provoqué par la différence de pression créée de part et d'autre de la membrane. De façon classique, on choisit des conditions de circulation du sang et du liquide de dialyse telles que la pression dans le compartiment du sang est supérieure à la pression dans le compartiment de liquide de dialyse. Ainsi, une fraction de liquide plasmatique passe par ultrafiltration au travers de la membrane 18, ce qui permet une meilleure épuration du sang.

Les conditions de pression requise pour provoquer l'ultrafiltration du sang peuvent être obtenues en faisant tourner au même débit les pompes de circulation 13 et 14 du liquide de dialyse, et en faisant fonctionner le dispositif d'injection. La pompe d'injection 23 aspirant dans le circuit de liquide de dialyse y provoque la dépression souhaitée. Dans ces conditions de fonctionnement, le liquide plasmatique ultrafiltré est remplacé par une même quantité de liquide de substitution injecté au patient.

Le liquide de dialyse aspiré dans la canalisation 9 par la pompe d'injection 23 est filtré par passage au travers de la membrane 18 du filtre principal 15. La membrane 18 retient les bactéries et les éléments pyrogènes du liquide de dialyse qui peut alors être injecté dans le circuit extracorporel de sang. Avant son injection dans le sang, le liquide de substitution passe au travers du filtre secondaire 24, qui, outre sa fonction spécifique décrite plus haut comme élément des moyens de vérification de l'intégrité du filtre principal, constitue également un organe de sécurité important en cas de rupture du filtre principal 15, en ce qu'il empêche l'injection au patient d'éléments bactériens et pyrogènes.

Selon l'invention, un tel incident sera détectée grâce aux informations provenant des capteurs de pression 25 et 26. En effet, dans le cas d'un défaut d'intégrité du filtre principal 15, les éléments indésirables non retenus par ce filtre seront arrêtés par le filtre secondaire 24 qui, étant donné sa surface réduite, aura tendance à se colmater rapidement. Un encrassement du filtre secondaire 24 se traduira, si le débit de la pompe d'injection 18 est maintenu constant, par une augmentation de la différence entre les pressions existant de part et d'autre du filtre secondaire 24.

Durant la séance d'hémodiafiltration, les capteurs de pression 25 et 26 fournissent à l'unité de commande 27 des signaux correspondant aux valeurs des pressions instantanées. L'unité de commande 27 calcule la différence des pressions et la compare à la valeur de seuil préalablement fixée et mise en mémoire. Si la différence des pressions atteint la valeur de seuil, I'unité de commande 27 déclenche les moyens d'alarme 28. L'unité de commande 27 peut en outre commander l'arrêt de la pompe d'injection 23. Comme mentionné plus haut, il est possible de choisir deux valeurs de seuil différentes : une première valeur correspondant à un léger encrassement du filtre secondaire 24 et servant de référence pour le déclenchement de l'alarme ; et seconde valeur de seuil correspondant à un encrassement plus important du filtre secondaire 24 et servant de référence pour la commande de l'arrêt de la pompe d'injection 23.

Il est possible également de ne pas comparer les valeurs instantanées de la différence des pressions à la valeur de seuil, mais de faire une moyenne, sur une certaine période, des valeurs de la différence des pressions et de comparer la valeur moyenne obtenue à la valeur de seuil. Ceci permet de tenir compte des variations de pression qui seraient dues, non pas à un colmatage du fitre secondaire 24, mais au fonctionnement de la pompe d'injection 23.

La différence des pressions de part et d'autre du microfiltre dépendant du débit fourni par la pompe d'injection 23, il est souhaitable de changer la valeur de seuil lorsque le débit de la pompe d'injection 23 change. Ceci peut être réalisé en fournissant à l'unité de commande 27 la relation existant entre la valeur de seuil et le débit de la pompe 23. Ainsi, I'unité de commande 27 recevant ultérieurement les informations relatives au débit de la pompe 23 pourra modifier la valeur seuil en fonction des changements de débit de la pompe.

Comme mentionné plus haut, avec le mode de fonctionnement qui vient d'être décrit, la quantité de liquide plasmatique soustraite au patient par ultrafiltration est compensée par une égale quantité de liquide de substitution injectée dans le circuit extracorporel de sang. Il est souvent souhaitable de faire perdre du poids au patient, c'est-à-dire de lui soutirer plus de liquide plasmatique qu'on ne lui injecte de liquide de substitution. Dans ce cas, il suffit de régler la pompe 14 de liquide de dialyse située en aval de l'hémodiafitre 1 de façon que son débit soit supérieur au débit de la pompe 13 de liquide de dialyse située en amont de l'hémodiafiltre 1. La différence entre les deux débits sera égale au débit de perte de poids.

L'invention n'est pas limitée au mode de réalisation qui vient d'être décrit. En particulier, il doit être clair que bien que le dispositif d'injection selon l'invention ait été décrit en combinaison avec une installation d'hémodiafiltration, ce dispositif peut être utilisé de façon totalement autonome. Au lieu d'être un liquide de dialyse préparé par un générateur de liquide de dialyse, le liquide utilisé sera préparé par exemple par mélange dosé d'eau et de solutions concentrées contenant les électrolytes et, éventuellement, les protéines souhaitées.

## Revendications

1. Dispositif d'injection d'un liquide stérile et apyrogène comprenant :
- au moins un filtre principal (15) ayant un premier et un second compartiments (16, 17) séparés par une membrane poreuse (18), le premier compartiment (16) ayant une entrée connectable à une source (11) de liquide à filtrer et le second compartiment (17) ayant un sortie connectée à une canalisation d'injection (22),
- des moyens (23) pour faire circuler le liquide au travers du filtre principal (15) et dans la canalisation d'injection (22), et
- des moyens (25, 26, 27) de vérification de l'intégrité du filtre principal (15),
caractérisé en ce que les moyens de vérification de l'intégrité du filtre principal (15) comprennent :
- un filtre secondaire (24) apte à retenir les bactéries et éléments pyrogènes disposé sur la canalisation d'injection (22), le filtre secondaire (24) ayant une surface de membrane suffisamment réduite pour qu'une rupture du filtre principal (15) ait pour conséquence un colmatage rapide du filtre secondaire (24) et
- des moyens (25, 26, 27) pour détecter un encrassement du filtre secondaire (24) pendant l'injection de liquide.

2. Dispositif selon la revendication 1, caractérisé en ce que les moyens pour détecter un encrassement du filtre secondaire (24) comprennent :
- des moyens (25, 26) pour mesurer une différence de pression existant de part et d'autre du filtre secondaire (24), et
- des moyens (27) pour comparer la différence de pression mesurée à une valeur de seuil.

3. Dispositif d'injection selon la revendication 2, caractérisé en ce qu'il comporte en outre des moyens (28) pour émettre une alarme lorsque la différence de pression mesurée atteint une valeur de seuil.

4. Dispositif d'injection selon une des revendications 1 à 3, caractérisé en ce que la source de liquide est un générateur de liquide de dialyse (11).

5. Dispositif d'injection selon une des revendications 1 à 4, caractérisé en ce que la canalisation d'injection (22) est connectable à un circuit de circulation extracorporelle de sang.

6. Dispositif d'injection selon une des revendications 1 à 5, caractérisé en ce que le premier compartiment (16) du filtre principal (15) comporte une sortie connectable à une canalisation d'évacuation pour permettre un nettoyage de la membrane (18), pendant l'injection, par circulation de liquide à filtrer tangentiellement à la membrane (18) au travers du premier compartiment.

7. Dispositif d'injection selon une des revendications 2 à 6, caractérisé en ce que le filtre secondaire (24) a une porosité inférieure à environ 0,45 µm.

8. Dispositif d'injection selon la revendication 7, caractérisé en ce que le filtre secondaire (24) a une porosité comprise entre environ 0,20 µm et environ 0,24 µm.

9. Procédé de contrôle de l'intégrité d'un filtre principal (15) utilisé pour rendre stérile et apyrogène un liquide destiné à être injecté en continu, le filtre principal (15) ayant un premier et un second compartiments (16, 17) séparés par une membrane poreuse (18) et le premier compartiment (16) ayant une entrée connectable à une source de liquide à filtrer (11) et le second compartiment (17) ayant un sortie connectée à une canalisation d'injection (22), caractérisé en ce qu'il comprend les étapes de :
- placer un filtre secondaire (24) sur la conduite d'injection (22), la surface de la membrane du titre secondaire (24) étant choisie suffisamment réduite pour qu'une rupture du filtre principal (15) ait pour conséquence un colmatage rapide du filtre secondaire (24), et
- mesurer dans la ligne d'injection, au cours de l'injection, la valeur d'un paramètre susceptible d'être influencé par un encrassement du filtre secondaire (24).

10. Procédé selon la revendication 9, caractérisé en ce que le paramètre est la différence des pressions existant de part et d'autre du filtre secondaire (24).

11. Procédé selon une des revendications 9 et 10, caractérisé en ce qu'il comporte en outre l'étape de comparer la valeur mesurée du paramètre à une valeur de seuil.

12. Procédé selon la revendication 11, caractérisé en ce que la valeur de seuil est choisie en fonction d'au moins une des caractéristiques suivantes du liquide d'injection : débit, température.

13. Procédé selon la revendication 11, caractérisé en ce que la valeur de seuil est choisie sensiblement égale à la valeur du paramètre mesurée au début de l'injection du liquide.

14. Procédé selon une des revendications 9 à 13, caractérisé en ce qu'il comporte en outre l'étape d'émettre une alarme lorsque la valeur mesurée du paramètre atteint une valeur de seuil.

15. Procédé selon une des revendications 9 à 14, caractérisé en ce qu'il comporte en outre l'étape d'arrêter l'injection lorsque la valeur mesurée du paramètre atteint une valeur de seuil.

## Claims

1. Device for injection of a sterile and pyrogen free fluid, comprising:
- at least one main filter (15) having a first and a second compartment (16, 17) which are separated by a porous membrane (18), the first compartment (16) having an inlet which can be connected to a source (11) of fluid to be filtered , and the second compartment (17) having an outlet connected to an injection channel (22),
- means (23) for circulating the fluid through the main filter (15) and into the injection channel (22), and
- means (25, 26, 27) for checking the integrity of the main filter (15),
characterized in that the means for checking the integrity of the main filter (15) comprises:
- a secondary filter (24) which is able to hold back the bacteria and pyrogenic elements and is arranged on the injection channel (22), the secondary filter (24) having a membrane surface small enough to ensure that a rupture of the main filter (15) results in a rapid clogging of the secondary filter (24), and
- means (25, 26, 27) for detecting a clogging of the secondary filter (24) during the injection of fluid.

2. Device according to claim 1, characterized in that the means for detecting a clogging of the secondary filter (24) comprises:
- means (25, 26) for measuring a difference in pressure existing on each side of the secondary filter (24), and
- means (27) for comparing the difference in pressure measured with a threshold value.

3. Injection device according to claim 2, characterized in that it further comprises means (28) for emitting an alarm when the pressure difference measured reaches a threshold value.

4. Injection device according to one of the claims 1 to 3, characterized in that the source of fluid is a dialysis fluid generator (11).

5. Injection device according to one of the claims 1 to 4, characterized in that the injection line (22) can be connected to an extracorporeal blood circuit.

6. Injection device according to one of the claims 1 to 5, characterized in that the first compartment (16) of the main filter (15) comprises an outlet which can be connected to a discharge channel in order to permit a cleaning of the membrane (18), during the injection, by fluid to be filtered circulating tangentially to the membrane (18) through the first compartment.

7. Injection device according to one of the claims 2 to 6, characterized in that the secondary filter (24) has a porosity of less than approximately 0,45 µm.

8. Injection device according to claim 7, characterized in that the secondary filter (24) has a porosity of between approximately 0,20 µm and approximately 0,24 µm.

9. Method for checking the integrity of a main filter (15) used to make sterile and pyrogen free a fluid intended to be injected continuously, the main filter (15) having a first and a second compartment (16, 17) which are separated by a porous membrane (18), and the first compartment (16) having an inlet which can be connected to a source of fluid to be filtered (11), and the second compartment (17) having an outlet connected to an injection channel (22), characterized in that it comprises the steps of:
- placing a secondary filter (24) on the injection conduit (22), the membrane surface of the secondary filter (24) being chosen small enough to ensure that a rupture of the main filter (15) results in a rapid clogging of the secondary filter (24), and
- measuring in the injection line, during the injection, the value of a parameter susceptible of being influenced by a clogging of the secondary filter (24).

10. Method according to claim 9, characterized in that the parameter is the difference in the pressures existing on each side of the secondary filter (24).

11. Method according to one of the claims 9 and 10, characterized in that it further comprises the step of comparing the measured value of the parameter with a threshold value.

12. Method according to claim 11, characterized in that the threshold value is chosen as a function of at least one of the following characteristics of the injection fluid: flow rate, temperature.

13. Method according to claim 11, characterized in that the threshold value is chosen substantially equal to the value of the parameter measured at the start of the injection of the fluid.

14. Method according to one of the claims 9 to 13, characterized in that it further comprises the step of emitting an alarm when the measured value of the parameter reaches a threshold value.

15. Method according to one of the claims 9 and 14, characterized in that it further comprises the step of stopping the injection when the measured value of the parameter reaches a threshold value.

## Patentansprüche

1. Vorrichtung zur Injektion einer sterilen und pyrogenfreien Flüssigkeit, die folgendes umfaßt:
- mindestens ein Hauptfilter (15) mit einem ersten und einem zweiten Abteil (16, 17), die durch eine poröse Membran (18) voneinander getrennt sind, wobei das erste Abteil (16) einen mit einer Quelle (11) von zu filtrierender Flüssigkeit verbindbaren Eingang und das zweite Abtei (17) einen mit einer Injektionsleitung (22) verbundenen Ausgang aufweist;
- Mittel (23) zur Zirkulierung der Flüssigkeit durch das Hauptfilter (15) und in die Injektionsleitung (22), und
- Mittel (25, 26, 27) zur Überprüfung der Unversehrtheit des Hauptfilters (15),
dadurch gekennzeichnet, daß die Mittel zur Überprüfung der Unversehrtheit des Hauptfilters (15) folgendes umfassen:
- ein zur Rückhaltung von Bakterien und Pyrogenen geeignetes und an der Injektionsleitung angeordnetes Sekundärfilter (24), dessen Membranfläche klein genug ist, daß ein Riß des Hauptfilters (15) das schnelle Verstopfen des Sekundärfilters (24) zur Folge hat, und
- Mittel (25, 26, 27) zur Erfassung eines Zusetzens des Sekundärfilters (24), während der Injektion von Flüssigkeit.

2. Vorrichtung nach Anspruch 1, dadurch gekennzeichnet, daß die Mittel zur Erfassung eines Zusetzens des Sekundärfilters (24) folgendes umfassen:
- Mittel (25, 26) zur Messung eines Druckunterschieds, der auf beiden Seiten des Sekundärfilters (24) vorliegt, und
- Mittel (27) zum Vergleichen des gemessenen Druckunterschieds mit einem Schwellenwert.

3. Injektionsvorrichtung nach Anspruch 2, dadurch gekennzeichnet, daß sie außerdem Mittel (28) zur Ausgabe eines Alarms umfaßt, wenn der gemessene Druckunterschied einen Schwellenwert erreicht.

4. Injektionsvorrichtung nach einem der Ansprüche 1 bis 3, dadurch gekennzeichnet, daß es sich bei der Flüssigkeitsquelle um eine Dialyseflüssigkeitserzeugung (11) handelt.

5. Injektionsvorrichtung nach einem der Ansprüche 1 bis 4, dadurch gekennzeichnet, daß die Injektionsleitung (22) mit einem extrakorporalen Blutkreislauf verbunden werden kann.

6. Injektionsvorrichtung nach einem der Ansprüche 1 bis 5, dadurch gekennzeichnet, daß das erste Abteil (16) des Hauptfilters (15) einen mit einer Abflußleitung verbindbaren Ausgang hat, so daß die Membran (18) während der Injektion durch das die Membran (18) tangierende Zirkulieren von zu filtrierender Flüssigkeit durch das erste Abteil gereinigt werden kann.

7. Injektionsvorrichtung nach einem der Ansprüche 2 bis 6, dadurch gekennzeichnet, daß das Sekundärfilter (24) eine Porosität von weniger als etwa 0,45 µm hat.

8. Injektionsvorrichtung nach Anspruch 7, dadurch gekennzeichnet, daß das Sekundärfilter (24) eine Porosität von etwa 0,20 µm bis etwa 0,24 µm hat.

9. Verfahren zur Kontrolle der Unversehrtheit eines Hauptfilters (15), das dazu verwendet wird, eine Flüssigkeit, die kontinuierlich injiziert werden soll, steril zu machen und von Pyrogenen zu befreien, wobei das Hauptfilter (15) ein erstes und ein zweites Abteil (16, 17) aufweist, die durch eine poröse Membran (18) voneinander getrennt sind, und wobei das erste Abteil (16) einen mit einer Quelle von zu filtrierender Flüssigkeit (11) verbindbaren Eingang und das zweite Abteil (17) einen mit einer Injektionsleitung (22) verbundenen Ausgang aufweist, gekennzeichnet durch die folgenden Schritte:
- man plaziert ein Sekundärfilter (24) in die Injektionsleitung (22), wobei die Oberfläche der Membran des Sekundärfilters (24) klein genug gewählt wird, daß ein Riß des Hauptfilters (15) ein schnelles Verstopfen des Sekundärfilters (24) zur Folge hat, und
- man mißt während der Injektion in der Injektionsleitung den Wert eines Parameters, der von einem Zusetzen des Sekundärfilters (24) beeinflußt werden kann.

10. Verfahren nach Anspruch 9, dadurch gekennzeichnet, daß der Parameter der Druckunterschied ist, der auf beiden Seiten des Sekundärfilters (24) vorliegt.

11. Verfahren nach Anspruch 9 oder 10, dadurch gekennzeichnet, daß es weiterhin das Vergleichen des gemessenen Werts des Parameters mit einem Schwellenwert umfaßt.

12. Verfahren nach Anspruch 11, dadurch gekennzeichnet, daß der Schwellenwert in Abhängigkeit von mindestens einem der folgenden Merkmale der Injektionsflüssigkeit, nämlich Durchflußmenge und Temperatur, gewählt wird.

13. Verfahren nach Anspruch 11, dadurch gekennzeichnet, daß der Schwellenwert so gewählt wird, daß er im wesentlichen gleich dem Wert des zu Beginn der Injektion der Flüssigkeit gemessenen Parameters ist.

14. Verfahren nach einem der Ansprüche 9 bis 13, dadurch gekennzeichnet, daß es weiterhin die Ausgabe eines Alarms umfaßt, wenn der gemessene Wert des Parameters einen Schwellenwert erreicht.

15. Verfahren nach einem der Ansprüche 9 bis 14, dadurch gekennzeichnet, daß es weiterhin das Anhalten der Injektion umfaßt, wenn der gemessene Wert des Parameters einen Schwellenwert erreicht.
